# EUROPÄISCHE PATENTSCHRIFT

(11) **EP 3 122 314 B1**
(45) Veröffentlichungstag und Bekanntmachung des Hinweises auf die Patenterteilung: **06.05.2020**
(21) Anmeldenummer: 15709933.4
(22) Anmeldetag: 13.03.2015
(51) Int. Cl.: A61K 8/34, A61Q 5/00, A61Q 5/06, A61Q 19/00, A61Q 19/10

(54) **KONSERVIERUNGSMITTELMISCHUNGEN UND DAMIT STABILISIERTE POLYMERLÖSUNGEN**
CONSERVATION AGENT MIXTURES AND POLYMER SOLUTIONS STABILISED WITH THE SAME
MÉLANGES DE MOYENS DE CONSERVATION ET SOLUTIONS POLYMÈRES AINSI STABILISÉES

(30) Priorität: 25.03.2014 EP 14161479
(43) Veröffentlichungstag der Anmeldung: 01.02.2017
(73) Patentinhaber: BASF SE, 67056 Ludwigshafen am Rhein (DE)
(72) Erfinder: POTZOLLI, Bernd de, 67098 Bad Dürkheim (DE); RAUSCH, Monika, 67125 Dannstadt-Schauernheim (DE); JUHKASON, Karin, 67150 Niederkirchen (DE)
(74) Vertreter: BASF IP Association
(86) Internationale Anmeldenummer: PCT/EP2015/055291
(87) Internationale Veröffentlichungsnummer: WO 2015/144458

(56) Entgegenhaltungen:
- WO-A1-2008/071027
- WO-A1-2014/032989
- DE-A1- 4 026 756
- JP-A- H1 053 510
- US-A1- 2005 228 032
- DATABASE GNPD [Online] MINTEL; 28. Februar 2014 (2014-02-28), "Eye Cream", XP002726539, Database accession no. 2265088

## Beschreibung

Die vorliegende Erfindung betrifft spezifische biozid wirkende Konservierungsmittelmischungen und wässrige Polymerlösungen, die mit diesen Konservierungsmittelmischungen oder durch Einmischung der genannten biozid wirkenden Einzelkomponenten stabilisiert sind und ihre Verwendung in kosmetischen Zubereitungen, wie Haut- und Haarpflegemitteln, sowie Haarstylingprodukten und in Produkten für den Home Care Bereich.

Das Wachstum von Mikroorganismen wie Bakterien und Viren sowie Pilzen und Hefen in Polymerlösungen, insbesondere wässrigen Polymerlösungen, ist in vieler Hinsicht zu vermeiden. Mikroorganismen in wässrigen Polymerlösungen führen zu gesundheitsschädlichen Verkeimungen und stellen somit ein hohes Hygienerisiko dar, insbesondere wenn die Polymerlösungen in kosmetische Produkte, beispielsweise als Bindemittel, Verdicker oder Filmbildner, eingearbeitet werden.

Es ist bekannt, wässrige Polymerlösungen mit Bioziden bzw. anti-mikrobiellen Verbindungen als Konservierungsmittel gegen störende Verkeimungen zu stabilisieren. Sie werden als Additive in die Polymerlösung eingebracht. Es hat sich allerdings herausgestellt, dass einige der bekannten Konservierungsmittel kanzerogen oder sensibilisierend sind und nicht für den Einsatz in kosmetischen Zubereitungen geeignet sind. So ist seit einiger Zeit bekannt, dass 2-Methyl-isothiazolin-3-on (MIT) als sehr häufig eingesetztes Konservierungsmittel von wässrigen Polymerlösungen sensibilisierend und damit gesundheitsschädlich ist.

Nachteile alternativer Additive, die weltweit zugelassen sind, bestehen darin, dass eine mangelnde Produktstabilität zu verzeichnen ist. Somit kommt es sehr häufig zu Verfärbungen, Trübungen, Phasentrennungen, negativen geruchlichen Veränderungen und nicht ausreichender mikrobiologischer Wirksamkeit in den damit versetzten Polymerlösungen.

Es ist daher Aufgabe der vorliegenden Erfindung, unerwünschte Verkeimungen in wässrigen Polymerlösungen zu vermeiden, wobei gute Produktstabilitäten erhalten bleiben. Es ist weiterhin Aufgabe der vorliegenden Erfindung, eine Technologie zu entwickeln, mit der kosmetische Produkte unbedenklich hinsichtlich ihrer Inhaltsstoffe hergestellt werden können.

Diese Aufgaben sind mit der vorliegenden Erfindung gelöst worden. Relevante Dokumente zum Stand der Technik sind Mintel Record ID 2265088 (XP 2726539, Eye Cream); Patentschrift DE 4026756 (Konservierungsmittel und deren Verwendung); und Patentschrift WO 2008/071027 (Compositions).

Die vorliegende Erfindung betrifft wässrige MIT-freie Polymerlösungen, gemäß vorliegendem Anspruch 1 die mit einer Konservierungsmittelmischung aus Phenoxyethanol und mindestens einem weiteren Alkoholen stabilisiert sind.

Gegenstand der vorliegenden Erfindung sind ebenfalls die in/aus den stabilisierten wässrigen Polymerlösungen enthaltenden/erhaltenen Polymere. Die Erfindung wird durch die Ansprüche definiert.

Die Erfindung betrifft weiterhin die Verwendung der wässrigen Polymerlösung in kosmetischen Zubereitungen, wie Haut- und Haarpflegemittel, Haarstylingprodukte, Produkte der dekorativen Kosmetik und kosmetischen Reinigung, z. B. Shampoos und Duschgele, sowie Flüssigseifen.

Die Erfindung betrifft außerdem MIT-freie Konservierungsmittelmischungen zur Stabilisierung von wässrigen Polymerlösungen.

Die Unteransprüche betreffen bevorzugte Ausführungsformen der erfindungsgemäßen wässrigen Polymerlösung, ihrer Polymere sowie der Verwendung.

Die Mengen für die Bestandteile der Konservierungsmittelmischung beziehen sich auf 100 Gew.-% wässrige Polymerlösung.

Die erfindungsgemäße wässrige Polymerlösung enthält eine Konservierungsmittelmischung, die in ausgewogener niedriger Dosierung Phenoxyethanol und mindestens einen weiteren Alkohol als biozid wirkende Wirkstoffe aufweist. Diese spezifischen Konservierungsmittelmischungen sind frei von 2-Methyl-isothiazolin-3-on (MIT), einem nachweislich gesundheitsschädlichen Konservierungsmittel. Des Weiteren enthalten die Konservierungsmittelmischungen kein Paraben und Polyaminopropylbiguanid.

Es war festgestellt worden, dass wässrige Polymerlösungen, die nur einen Bestandteil der Konservierungsmittelmischung enthalten, das heißt, nur Phenoxyethanol oder einen Alkohol, keinen ausreichenden stabilisierenden Effekt selbst in vielfach höherer Konzentration zeigen. Daher war es umso überraschender, dass die Konservierungsmittelmischungen mit Phenoxyethanol in Kombination mit einem Alkohol in einem niedrigen Konzentrationsbereich der einzelnen Komponenten unerwartete Effekte bei der mikrobiellen Stabilisierung der erfindungsgemäßen wässrigen Polymerlösungen zeigen. Es erwies sich weiterhin besonders vorteilhaft, dass die die erfindungsgemäßen Konservierungsmittel enthaltenden Polymerlösungen stabil, klar, farblos, farbstabil und geruchsakzeptabel (keine störenden Fremdgerüche) sind und keine Phasentrennung zeigen. Außerdem sind bei den verschiedenen Anwendungen keine Nachteile, wie z.B. Trübungen und Verfärbungen und keine Erniedrigung der Viskositäten bei beispielsweise Gelen und Schäumen etc. zu verzeichnen.

Ein weiterer Vorteil der vorliegenden Erfindung ist darin zu sehen, dass die erfindungsgemäßen Polymerlösungen durch die geringe Konzentration der Konservierungsmittel äußerst wirtschaftlich im Sinne von kostengünstig stabilisiert werden können.

Die synergistisch verstärkende Wirksamkeit des Phenoxyethanols mit mindestens einem weiteren Alkohol als Booster richtet sich gegen verschiedene Keime bzw. Keimmischungen, wie beispielsweise gram-positive und gram-negative Bakterien, Hefen und Schimmel. Dabei bleibt die Produktstabilität der erfindungsgemäßen wässrigen Polymerlösungen erhalten. Der Booster-Effekt kann aber auch vom Phenoxyethanol ausgehen. Dieses Ergebnis war nicht vorhersehbar, insbesondere im Hinblick darauf, dass die einzelnen Komponenten in der Konservierungsmittelmischung in wesentlich niedrigen Konzentrationen in den Polymerlösungen im Vergleich zu herkömmlichen Konservierungsmitteln vorhanden sind. In einer bevorzugten Ausführungsform der vorliegenden Erfindung sind die Alkohole der Konservierungsmittelmischung aus der Gruppe ein-, zwei- und dreiwertige Alkohole sowie Mischungen daraus ausgewählt. Bevorzugt handelt sich hierbei um aliphatische und aromatische Alkohole, wobei die aliphatischen Ketten gerad- oder verzweigtkettig sein können.

Ebenfalls offenbart sind wässrige Polymerlösungen die mit Konservierungsmittelmischungen stabilisiert sind, die, neben Phenoxyethanol, Alkohole aus der Gruppe Ethylhexylglycerin, 1,3-Propandiol, 1,2-Pentandiol, 1,2-Octandiol, 3-Phenylpropanol, 2-Phenylethanol, Ethanol, Isopropanol und 1,2-Decandiol sowie Mischungen daraus enthalten. Ganz besonders bevorzugt sind 3-Phenylpropanol, 1,3-Propandiol, 1,2-Pentandiol, 1,2-Octandiol, 1,2-Decandiol, Ethanol, Isopropanol und Mischungen daraus.
Die Konzentration der Konservierungsmittelmischung in der wässrigen Polymerlösung liegt in einem Bereich von 0,05 bis 1,40 Gew.-%, vorzugsweise in einem Bereich von 0,10 bis 0,65 Gew.-%, und ganz besonders bevorzugt in einem Bereich von 0,10 bis 0,40 Gew.-%.
Die vorliegende Erfindung beschreibt ebenfalls Konservierungsmittelmischungen, die für die Stabilisierung von wässrigen Polymerlösungen eingesetzt werden.

Eine erfindungsgemäße Konservierungsmittelmischung (Mischung A) weist als biozid wirkende Wirkstoffe folgende Bestandteile auf:
25 bis 50 Gew.-% einer Mischung aus 50% 3-Phenylpropanol, 25% 1,3-Propandiol und 25% 1,2-Octandiol und
50 bis 75 Gew.-% Phenoxyethanol,
bezogen auf 100 Gew.-% Mischung.

Eine weitere erfindungsgemäße Konservierungsmittelmischung (Mischung B) ist wie folgt zusammengesetzt:
20 bis 57 Gew.-% einer Mischung aus 50% 1,2-Pentandiol, 30% 1,2-Octandiol und 20% 1,2-Decandiol und
43 bis 80 Gew.-% Phenoxyethanol
bezogen auf 100 Gew.-% Mischung.

Die wässrige Polymerlösung der vorliegenden Erfindung wird mikrobiell durch die spezifischen Konservierungsmittelmischungen oder durch Zusatz der Einzelkomponenten stabilisiert. Um den beanspruchten Konzentrationsbereich in der wässrigen Polymerlösung zu erreichen, wird die Mischung A in einer Menge von 0,10 bis 1,40 Gew.-% eingesetzt. Die Mischung A wird in einer Menge von 0,2 bis 0,8 Gew.-% eingesetzt, um eine bevorzugte wässrige Polymerlösung zu erhalten. Die Mischung A wird in einer Menge von 0,40 bis 0,60 Gew.-% eingesetzt. um eine ganz bevorzugte wässrige Polymerlösung herzustellen. Die Mischung B wird in einer Menge von 0.20 - 1.4 Gew.-% eingesetzt. Ein bevorzugter Bereich ist 0,25 bis 0,75 Gew. in der Lösung, wobei 0,30 bis 0,60 Gew.-% besonders bevorzugt ist.

Der Begriff "Polymer" umfasst beispielsweise lineare, wasserlöslich verzweigte oder wasserunlöslich lineare und verzweigte Polymere. Der Begriff "wasserunlöslich verzweigtes Polymer" umfasst auch die sogenannten Popcorn-Polymere, die im Englischen als "proliferous polymers" oder wie bei Polyvinylpyrrolidon als PVPP bezeichnet werden. "Verzweigt", "verzweigend", "vernetzt", "vernetzend" wird im Rahmen dieser Erfindung austauschbar verwendet und bedeutet ein Polymer, das mindestens eine Verzweigungsstelle aufweist.

"Polymer" umfasst auch die Copolymere, Graft-Homo- oder Graft-co-Polymere, die jeweils als lineare oder löslich-vernetzte, insbesondere wasserlöslich vernetzte, oder unlöslichvernetzte, insbesondere wasserunlöslich-vernetzte, Polymere vorliegen können.

"Polymer" kann in jedweder Form vorliegen, etwa als Di- oder Multi-Blockpolymere, wie auch in Stern-, Bürsten- oder hyperverzweigter Form oder als Dendrimer vorliegen.

Erfindungsgemäße Polymere werden in den Ansprüchen definiert. Ebenfalls offenbart werden Polymere enthaltend ein oder mehrere Monomere a), gegebenenfalls ein oder mehrere Monomere b) sowie gegebenenfalls ein oder mehrere vernetzende Monomere c), das heißt, sie sind durch Polymerisation der genannten Monomeren erhalten worden und können noch Restmengen der Monomeren enthalten.

Monomere a) sind ausgewählt aus:
N-Vinyllactame wie N-Vinylpyrrolidon, N-Vinylpiperidon, N-Vinylcaprolactam, deren mit C1 bis C8-Alkyl-Gruppen-substiutierten Derivate wie 3-Methyl-, 4-Methyl- oder 5-Methyl-N-Vinylpyrrolidon und Mischungen davon, Vinylacetat und Mischungen von N-Vinyllactamen, bevorzugt mit N-Vinylpyrrolidon, Vinylacetat sowie Acylate und Methacylate einschließlich Säuren davon und deren Monomermischungen mit N-Vinyllactamen und/oder Vinylimidazolen und/oder quaternisierten Vinylimidazolen sowie Diisocyanate und Diamine zur Bildung von Polyurethanen mit oder ohne endständige EO-/PO-Fettalkohole, N-Vinylamide wie N-Vinylformamid und dessen nach der Polymerisation durch Hydrolyse erhältliches N-Vinylamin, N-Vinyl-N-methylacetamid.

Amine wie N-Vinyl- oder Allyl-substituierte heterocyclische Verbindungen, bevorzugt N-Vinylpyridin, oder N-Allylpyridin, N-Vinylimidazole, die auch in 2-, 4- oder 5-Position mit C1-C4-Alkyl, insbesondere Methyl- oder Phenyl-Resten substituiert sein können, wie 1-Vinylimidazol, 1-Vinyl-2-methyl(ethyl)vinylimidazol sowie deren quaternisierte Analoga wie 3-Methyl-1-vinylimidazoliumchlorid, 3-Methyl-1-vinylimidazoliummethyl(ethyl)sulfat, N- C1- bis C24-Alkyl-substituierte Diallylamine oder deren quaternisierte Analoga wie Diallylammoniumchlorid oder Diallyldimethylammoniumchlorid und Diamine. Erfindungsgemäße Polymere enthalten dabei bevorzugt wenigstens ein N-Vinyllactam-Monomer.

Weitere Monomere a) können Polyacrylamide, Polymethacrylamide, Polyacrylate, Polymethacrylate, neutrale Polyacrylsäuren, und neutrale Polymethacrylsäuren sein.

Erfindungsgemäße Polymere können Homopolymere als auch Copolymere aus zwei oder mehreren der Monomere a) sein, beispielsweise Copolymere aus N-Vinylpyrrolidon und N-Vinylimidazol, Copolymere aus N-Vinylpyrrolidon und N-Vinylformamid, Copolymere aus N-Vinylpyrrolidon und N-Vinylcaprolactam, Copolymere aus N-Vinylpyrrolidon, N-Vinylcaprolactam und N-Vinylimidazol oder Copolymere aus N-Vinylpyrrolidon und N-Vinylimidazol und quaternisierte N-Vinylimidazole.

Bevorzugte Monomere a) sind Vinyllactame wie N-Vinylpyrrolidon, 3-Methyl-N-vinylpyrrolidon, 4-Methyl-N-vinylpyrrolidon, 5-Methyl-N-vinylpyrrolidon, N-Vinylpiperidon und N-Vinylcaprolactam, Vinylacetat sowie der durch Hydrolyse nach der Polymerisation erhältliche Vinylalkohol, Vinylamide wie Vinylformamid sowie das durch Hydrolyse nach der Polymerisation erhältliche Vinylamin, N-Vinylimidazol, 1-Vinyl-3-methylimidazolium Chlorid, 1-Vinyl-3-methylimidazolium Sulfat, und Vinylmethylamid sowie deren Derivate.

Weitere bevorzugte Monomere a) sind Acylate und Methacylate und ihre Säuren sowie Diamine.

Ganz besonders bevorzugte Monomere a) sind N-Vinylpyrrolidon, N-Vinylcaprolactam, Vinylacetat, Vinylformamid sowie das durch Hydrolyse nach der Polymerisation erhältliche Vinylamin sowie N-Vinylimidazol.

Als Monomere b) kommen alle Monomere in Betracht, die in WO 2010/072640 A1 als "Monomer b)" auf den Seite 6, ab Zeile 8, bis Seite 8, Zeile 17, genannt sind.

Bevorzugte Monomere b) sind Acrylate, Acrylsäure, Alkylacrylate, Alkylacrylsäuren, Methacrylate, Methacrylsäure, Alkylmethacrylate, Alkylmethacrylsäuren, Maleinsäure, Maleinsäureanhydrid, Isopropylmethacrylamid, Acrylamid, Methacrylamid, 2-Hydroxyethylacrylamid und 2-Hydroxyethylmethacrylamid, ferner, Vinylester aliphatischer C2-C18-Carbonsäuren wie Vinylacetat sowie der durch Hydrolyse nach der Polymerisation erhältliche Vinylalkohol, Vinylpropionat, Vinylbutyrat, Vinyllaurat, Vinylstearat, Vinylneodecanoat VEOVA 9 und VEOVA 10, ferner Dimethylaminoethyl(meth)acrylat und Dimethylaminoethyl(meth)acrylamid und deren quaternierte Analoga sowie Diallyldimethylammoniumchlorid und Diisocyanate, sowie Styrol und Styrolderivate.

Ganz besonders bevorzugte Monomere b) sind Methacrylamid, Vinylacetat sowie der durch Hydrolyse nach der Polymerisation erhältliche Vinylalkohol, Vinylpropionat, Vinylneodecanoat VEOVA 9 und VEOVA 10, Dimethylaminoethyl(meth)acrylat oder Dimethylaminoethyl(meth)acrylamid oder deren quaternierte Analoga sowie Diallyldimethylammoniumchlorid.

Polymere, die Copolymere sind und Monomere b) enthalten, können eines oder mehrere der Monomere b) enthalten. Üblicherweise sind jedoch nicht mehr als fünf verschiedene Monomere b) in einem Copolymer enthalten.

Zu den bevorzugten Polymeren zählen des weiteren Copolymere, die ein oder mehrere Monomere a) und/oder ein oder mehrere Monomere b) enthalten.

Als vernetztende Monomere c) ("Vernetzer") kommen in Betracht:
Vernetzende Monomere c) sind beispielsweise in WO2009/024457 auf Seite 7, Zeile 1, bis Seite 9, Zeile 2, beschrieben.

Besonders bevorzugt als vernetzende Monomere c) sind Pentaerythrittriallylether, Methylenbis-acrylamid, N,N'-Divinylethylenharnstoff, Divinylbenzol, Ethylen-bis-N-vinylpyrrolidon, 3-Vinyl-N-vinylpyrrolidon, 4-Vinyl-N-vinylpyrrolidon, 5-Vinyl-N-vinylpyrrolidon, Allyl(meth)acrylat, Triallylamin und Acrylsäureester von Glykol, Butan-diol, Trimethylolpropan oder Glycerin sowie Acrylsäureester von mit Ethylenoxid und/oder Epichlorhydrin umgesetztem Glykol, Butandiol, Trimethylolpropan oder Glycerin.

Die Mengenanteile in Gewichtsprozent bezogen auf die Gesamtmasse des Polymeren betragen dabei für die Monomere a) üblicherweise mindestens 20, bevorzugt mindestens 30, besonders bevorzugt mindestens 50, insbesondere bevorzugt mindestens 60 Gewichtsprozent und ganz insbesondere bevorzugt bis zu 100 Gewichtsprozent wie beispielsweise Homopolymere aus 100% eines Monomeren a).

Die Mengenanteile in Gewichtsprozent bezogen auf die Gesamtmasse des Polymeren betragen dabei für die Monomere b) üblicherweise bis zu 80, bevorzugt bis zu 70, besonders bevorzugt bis zu 50, insbesondere bevorzugt bis zu 40 und ganz insbesondere bevorzugt weniger als 5 Gewichtsprozent und sind beispielsweise gar nicht im Polymer vorhanden.

Wenn das Polymer wasserlöslich vernetztes Polymer ist, betragen die Mengenanteile der vernetzenden Monomere c) in Gewichtsprozent bezogen auf die Gesamtmasse des Polymers üblicherweise 0,001 bis 20, bevorzugt 0,01 bis 10, besonders bevorzugt 0,05 bis 5 und insbesondere 0,1 bis 1 Gewichtsprozent.

Wenn das Polymer wasserunlöslich vernetztes Polymer wie etwa ein Popcornpolymer ist, betragen die Mengenanteile der vernetzenden Monomere c) in Gewichtsprozent bezogen auf die Gesamtmasse des Polymers üblicherweise 0,001 bis 10, bevorzugt 0,01 bis 5, besonders bevorzugt 0,1 bis 3 und insbesondere 0,5 bis 2 Gewichtsprozent.

Wird vernetzendes Monomer c) eingesetzt, so reduzieren sich die Gesamt-Mengenanteile von Monomer a) und gegebenenfalls Monomer b) entsprechend um die eingesetzte Menge an vernetzendem Monomer c).

Die zur Polymerisation eingesetzten Monomere a), b) und c) können unabhängig voneinander ein einzelnes oder Mischungen mehrere Monomerer a), Monomerer b) und/oder Monomerer c) sein, wobei der gemeinsame Mengenanteil der Monomere a), b) oder c) den jeweils dafür genannten Mengenanteil für Monomer a), für Monomer b) beziehungsweise für Monomer c) am Polymer ergibt.

Die Gesamtmengen an Monomer(en) a) plus Monomer(en) b) plus Monomer(en) c) addieren sich dabei immer zu 100 Gewichtsprozent.

Ein Vinyllactam-Polymer kann ein Homo- oder Copolymer sein enthaltend N-Vinyllactame wie N-Vinylpyrrolidon (VP) oder deren an 3, 4- oder 5-Position Methyl-substitutierten Derivate, N-Vinylpiperidon oder N-Vinylcaprolactam (VCap). Bevorzugt ist N-Vinylpyrrolidon, N-Vinylcaprolactam oder deren Mischung. Insbesondere bevorzugt ist N-Vinylpyrrolidon.
Bevorzugte Vinyllactam-Polymere sind Vinylpyrrolidon-Polymere wie Polyvinylpyrrolidone, Vinylpyrrolidon-Copolymere und Vinylpyrrolidon-Popcorn-Polymere.
Bevorzugte Polyvinylpyrrolidone sind Polymere mit K-Werten von 1 bis 150, vorzugsweise K10 bis K120, beispielsweise K12, K15, K 17, K25, K30, K60, K85, K90, K95, K100, K115 oder K120. Besonders bevorzugte PVP-Homopolymere weisen einen K-Wert von 12 bis 95 und insbesondere bevorzugt von 30 bis 90 auf, wie insbesondere K30, K60, K85 und K 90.

Bevorzugte Vinylpyrrolidon-Copolymere sind lineare, unvernetzte Copolymere mit N-Vinylcaprolactam (VCap), Vinylacetat (VAc), N-Vinylimidazol (VI), quaterniertem N-Vinylimidazol und/oder dessen Derivate und/oder deren Mischungen.

Besonders bevorzugte Copolymere sind Copolymere aus N-Vinylpyrrolidon (VP) mit Vinylacetat mit einem Gewichtsverhältnis VP/VAc von 20:80 bis 80:20, beispielsweise 30:70, 50:50, 60:40, 70:30, mit K-Werten von 10 bis 90, vorzugsweise von 15 bis 80 und insbesondere von 20 bis 60. Ganz besonders bevorzugte Copolymere aus N-Vinylpyrrolidon mit Vinylacetat weisen einen K-Wert von 22 bis 40 und ein Gewichtsverhältnis VP zu VAc von 50:50 bis 70:30 auf.

Ebenso bevorzugt sind Copolymere aus VP und VCap mit K-Werten von 10 bis 100, vorzugsweise von 12 bis 80 und insbesondere von 20 bis 75 sowie Gewichtsverhältnissen der Monomere VP zu VCap von 80:20 bis 20:80, bevorzugt von 70:30 bis 30:70, insbesondere bevorzugt von 60:40 bis 40:60 und beispielsweise auch 50:50.

Der K-Wert der Vinylpyrrolidon-Copolymere und der Polyvinylpyrrolidone (Fikentscher K-Wert; siehe etwa Bühler, "Polyvinylpyrrolidone - Excipient for Pharmaceuticals", Springer, 2005, Seite 40 bis 41) ist ein Maß für die Lösungsviskosität bei definierten Bedingungen. Damit ist er ein direktes Maß für die Molmasse. Verändert sich die Molmasse beispielsweise durch oxidative Prozesse, führt dies zu Molmassenaufbau (führt zur K-Wert-Erhöhung) oder zu Molmassenabbau (führt zur K-Wert-Erniedrigung) und so zu einer Veränderung des K-Werts. Verändert sich die Molmasse, so verändert sich entsprechend auch die Lösungsviskosität einer Lösung mit einem definierten Festgehalt.

Bevorzugte wässrige Polymerlösungen sind Polyvinylpyrrolidon-Lösungen, Polyvinylpyrrolidon/Polyvinylacetat-Lösungen, PVP/(VCap)/VI, quart. QVI-Lösungen und Polyurethan-Lösungen.

Die wässrigen Polymerlösungen werden nach herkömmlichen Verfahren hergestellt, zum Beispiel durch Einrühren der erfindungsgemäßen Konservierungsmittelmischungen oder durch direktes Einmischen der Einzelkomponenten der mindestens zwei Konservierungsmittel/Booster.

Die stabilisierten Polymere der Erfindung werden aus den wässrigen Lösungen auf üblichem Weg, beispielsweise durch Trocknung, wie Sprühtrocknung, Trocknung auf Kontaktflächen und Trocknung mittels Vakuum, hergestellt.

Erfindungsgemäß werden die mit der Konservierungsmittelmischung stabilisierten wässrigen Polymerlösungen und die Polymere daraus in kosmetischen Zubereitungen als beispielsweise Bindemittel, Verdicker oder Filmbildner eingesetzt. Beispiele für solche kosmetische Zubereitungen sind Haut- und Haarpflegemittel. Zu den Hautpflegemitteln zählen beispielsweise Cremes, Gele, Gelcremes, Wachse, Wachsgele, Gelwachse und Lotionen. Zu den Haarpflegemitteln zählen beispielsweise Cremes, Gele, Gelcremes, Wachse, Wachsgele, Gelwachse, Styling- und Conditionerschäume sowie Sprays (Pumpsprays und Aerosolsprays) und Lotionen. In diesem Zusammenhang sind auch Produkte zur kosmetischen Reinigung zu nennen, wozu Duschgels und Badegels zählen. Die mit den erfindungsgemäßen Konservierungsmittelmischungen stabilisierten wässrigen Polymerlösungen werden auch auf dem Gebiet Home Care mit Erfolg eingesetzt. Sie eignen sich daher für die Einarbeitung in Reinigungsmittel für den Haushalt, beispielsweise für harte Oberflächen.

Die erfindungsgemäßen stabilisierten wässrigen Polymerlösungen und die Polymere daraus können ebenfalls in der dekorativen Kosmetik eingesetzt werden. Dazu zählen beispielsweise Make-ups, Lippenstifte, Concealer und Mascaras.

Eine kosmetische Zubereitung liegt in der Regel als Gel, Wachs, Wachsgel, Gelwachs, Schaum, Spray oder Emulsion bzw. Dispersion vor und kann eine Anzahl weiterer kosmetischer Zusatzstoffe enthalten. Je nach Verwendung der erfindungsgemäßen Zubereitung müssen noch weitere Zusatzstoffe beigemischt werden, die ausgewählt sind aus der Gruppe, die gebildet wird von Emulgatoren, Tensiden, Perlglanzwachse, Stabilisatoren, Salz, Verdickungsmittel, Konsistenzgeber, anorganische und organische UV-Lichtschutzfilter, Selbstbräuner, Pigmente, Antioxidationsmittel, Hydrotope, biogene Wirkstoffe, Farbstoffe, übliche Konservierungsmittel bevorzugt Benzoesäure oder Zitronensäure, Feuchthaltemittel wie Glycerin, Ethanol, Propylenglykol, Antischuppenmittel, Quellmittel und Parfums. Als biogene Wirkstoffe sind dabei insbesondere Tocopherol, Tocopherolacetat, Tocopherolpalmitat, Desoxyribonucleinsäure, Coenzym Q10, Ascorbinsäure, Retinol- und Retinylderivate, Bisabolol, Allantoin, Phytantriol, Panthenol, AHA-Säuren, Aminosäuren, Ceramide, essentielle Öle, Hyaloronsäure, Creatin, Proteinhydrolysate, Pflanzenextrakte, Peptide und Vitaminkomplexe bevorzugt.

Ein weiterer Aspekt der vorliegenden Erfindung liegt in der Verwendung der erfindungsgemäß stabilisierten wässrigen Polymerlösung oder der Polymere daraus als Konditioniermittel in Haarpflegemitteln. Die erfindungsgemäß stabilisierte wässrige Lösung und die Polymere daraus können in Haarshampoos, Haarpflegemitteln, Haarlotionen, Haarkuren, Haargel, Haarschäumen, Conditionern, Haarwässern und Haarstylingprodukten, wie Styling- und Conditionerschäumen, Haargelen, Haarschäumen, Haarfestigern (in z.B. Sprays und Lotionen) und Haarwachsen eingearbeitet werden Die Verwendung in Haarshampoo macht den Einsatz weiterer Tenside sowie Hilfs- und Zusatzstoffe nötig. Die Zugabe weiterer üblicher Zusatzstoffe richtet sich nach dem jeweiligen Produkt.

Die erfindungsgemäßen Konservierungsmittelmischungen eignen sich aufgrund ihrer bioziden Eigenschaften und ihres Einsatzes in niedrigen Konzentrationen hervorragend zur Stabilisierung von wässrigen Polymerlösungen. Die Produktstabilität der stabilisierten Polymerlösungen bleibt über einen langen Zeitraum erhalten, das Aussehen und die Sensorik zeigen sich unverändert und es ergeben sich keine Farbveränderungen und Trübungen sowie Phasentrennungen der Lösungen. Die erfindungsgemäßen Polymerlösungen und auch die Polymere daraus zeigen eine außergewöhnlich gute mikrobielle Stabilität und können unbedenklich in kosmetische Produkte und Haarpflegemitteln sowie Haarstyling-Produkten eingearbeitet werden.

Die Erfindung wird anhand der folgenden Beispiele näher erläutert.

### Beispiele

Für die Ermittlung der Keimfreiheit der wässrigen Polymerlösungen wurden Keimbelastungstests durchgeführt, die den Anforderungen der Pharmakopoen für topische Applikationen (z.B. PhEur., USP und JP) erfüllen. Des Weiteren wurde der "Koko-Test" durchgeführt, der im Vergleich zu PhEur. bis zu sechsmalige Animpfungen mit Keimen vorsieht.

Der bestandene Keimbelastungstest nach Ph. Eur., 7th Edition mit den stabilisierten wässrigen Polymerlösungen zeigte nach 28 Tagen Keimfreiheit im Hinblick auf die getesteten Stämme Pseudomonas aeruginosa (ATCC 9027), Staphylococcus aureus (ATCC 6638), Candida albicans (ATCC 10231) und Aspergillus brasiliensis (ATCC 16404).

Die nachfolgende Tabelle 1 zeigt mit den Beispielen 1 bis 18 Polymerlösungen, die mit verschiedenen Konservierungsmittelmischungen in unterschiedlichen Konzentrationen stabilisiert sind. Wie zu erkennen ist, bestehen alle stabilisierten Polymerlösungen den Keimbelastungstest. Die Polymerlösungen der Beispiele 1 bis 15 enthalten die Konservierungsmittelmischung in sehr niedriger Konzentration im Bereich von 0,4 bis 0,8 Gew.-%, wobei die Produkte klar und wenig geruchsintensiv sind. Es sind keine Phasentrennungen zu verzeichnen. Es ist bemerkenswert, dass die wässrigen Luviskol K85/90-Lösungen mit Ethanol bzw. Isopropanol der Beispiele 16 bis 18 in Kombination mit höheren Anteilen an Phenoxyethanol in einer Konzentration im Bereich von 0,85 bis 1,35 Gew.-% wirkungsvoll stabilisiert werden können. Hier ist der boosternde Effekt von Ethanol oder Isopropanol auf das Phenoxyethanol deutlich zu erkennen.

Die Konservierungsmittelmischung ist die, die in den Ansprüchen festgelegt ist. Ebenfalls offenbart werden:
Das als a) verwendete Konservierungsmittel enthält Phenoxyethanol als einzelne Komponente.

Die als b) verwendete Konservierungsmittelmischung enthält die einzelnen Komponenten in folgenden Anteilen: 42 bis 48 % Phenylpropanol, 23 bis 27 % 1,2-Octandiol und 27 bis 33 % 1,3-Propandiol. Die Konservierungsmittelmischung c) enthält die einzelnen Komponenten wie folgt: 25 bis 50 % 1,2-Pentandiol, 25 bis 50 % 1,2-Octandiol und 10 bis 25 % 1,2-Decandiol. Die Konservierungsmittelmischung d) enthält 90% Phenoxyethanol und 10 % Ethylhexylglycerin. Das Konservierungsmittel e) ist entweder Ethanol oder Isopropanol.

**Tabelle 1**

| Beispiel | Polymer in wässriger Polymerlösung | Konservierungsmittelmischung | Konzentration der einzelnen Komponenten in der Polymerlsg. (Gew.-%) | Keimbelastungs test | Aussehen der stabilisierten Lösung |
|---|---|---|---|---|---|
| 1 | Luviset Clear* | a) 2-Phenoxyethanol | a) 0,10 | bestanden | fast klar |
| | | b) Phenylpropanol | b) 0,30 | | |
| | | 1,2-Octandiol | | | |
| | | 1,3-Propandiol | | | |
| 2 | Luviset Clear * | a) 2-Phenoxyethanol | a) 0,40 | bestanden | klar/fast klar |
| | | b) Phenylpropanol | b) 0,10 | | |
| | | 1,2-Octandiol | | | |
| | | 1,3-Propandiol | | | |
| 3 | Luviset Clear * | a) Phenoxyethanol | a) 0,1 | bestanden | fast klar, sehr leicht trüb |
| | | c) 1,2-Pentandiol | c) 0,45 | | |
| | | 1,2-Octandiol | | | |
| | | 1,2-Decandiol | | | |
| 4 | Luviset Clear * | a) Phenoxyethanol | a) 0,45 | bestanden | fast klar |
| | | c) 1,2-Pentandiol | c) 0,25 | | |
| | | 1,2-Octandiol | | | |
| | | 1,2-Decandiol) | | | |
| 5 | Luvigel Star ** | a) 2-Phenoyxethanol | a) 0,35 | bestanden | klar |
| | | b) Phenylpropanol | b) 0,35 | | |
| | | 1,2-Octandiol | | | |
| | | 1,3-Propandiol | | | |
| 6 | Luvigel Star ** | a) Phenoxyethanol | a) 0,2 | bestanden | klar |
| | | c) 1,2-Pentandiol | c) 0,6 | | |
| | | 1,2-Octandiol | | | |
| | | 1,2-Decandiol | | | |
| 7 | Luvigel Star ** | a) Phenoxyethanol | a) 0,55 | bestanden | klar |
| | | c) 1,2-Pentandiol | c) 0,25 | | |
| | | 1,2-Octandiol | | | |
| | | 1,2-Decandiol | | | |
| 8 | Luvigel Star ** | a) 2-Phenoyxethanol | a) 0,25 | bestanden | klar |
| | | b) Phenylpropanol | b) 0,55 | | |
| | | 1,2-Octandiol | | | |
| | | 1,3-Propandiol | | | |
| 9 | Luviquat Hold*** | a) Phenoxyethanol | a) 0,10 | bestanden | klar |
| | | b) Phenylpropanol | b) 0,30 | | |
| | | 1,2-Octandiol | | | |
| | | 1,3-Propandiol | | | |
| 10 | Luviquat Hold*** | a) Phenoxyethanol | a) 0,40 | bestanden | klar |
| | | b) Phenylpropanol | b) 0,10 | | |
| | | 1,2-Octandiol | | | |
| | | 1,3-Propandiol | | | |
| 11 | Luviquat Hold*** | a) Phenoxyethanol | a) 0,1 | bestanden | fast klar, sehr leicht trüb |
| | | c) 1,2-Pentandiol | c) 0,45 | | |
| | | 1,2-Octandiol | | | |
| | | 1,2-Decandiol | | | |
| 12 | Luviquat Hold*** | a) Phenoxyethanol | a) 0,45 | bestanden | fast klar |
| | | c) 1,2-Pentandiol | c) 0,25 | | |
| | | 1,2-Octandiol | | | |
| | | 1,2-Decandiol | | | |
| 13 | Luviskol | a) Phenoxyethanol | a) 0,15 | bestanden | klar |
| | K85/ | b) Phenylpropanol | b) 0,35 | | |
| | K90**** | 1,2-Octandiol | | | |
| | | 1,3-Propandiol | | | |
| 14 | Luviskol | a) Phenoxyethanol | a) 0,45 | bestanden | klar |
| | K85/ | b) Phenylpropanol | b) 0,25 | | |
| | K90**** | 1,2-Octandiol | | | |
| | | 1,3-Propandiol | | | |
| 15 | Luviskol | a) Phenoxyethanol | a) 0,50 | bestanden | klar/fast |
| | K85/ | c) 1,2-Pentandiol | c) 0,15 | | klar |
| | K90**** | 1,2-Octandiol | | | |
| | | 1,2-Decandiol | | | |
| 16 | Luviskol | d) Phenoxyethanol Ethylhexylglycerin | d) 0,75 | bestanden | klar |
| | K 85**** | e) Ethanol | e) 0,1 | | |
| 17 | Luviskol | d) Phenoxyethanol Ethylhexylglycerin | d) 1,00 | bestanden | klar |
| | K 85**** | e) Ethanol | e) 0,1 | | |
| 18 | Luviskol K90***** | d) Phenoxyethanol Ethylhexylglycerin | d) 1,10 e) 0,25 | bestanden | klar |
| | | e) Isopropanol | | | |

| | | | | | |
|---|---|---|---|---|---|
| * = Copolymer aus Vinylpyrrolidon, Methacrylamid und Vinylimidazol, 20 %ige wässrige Lösung ** = Polyurethan -39, 20 %ige wässrige Lösung *** = Polyquaternium -46, 20 %ige wässrige Lösung **** = Polyvinylpyrrolidon, 20 %ige wässrige Lösung Die Beispiele 1 bis 3, 6, 8 bis 11, 13 und 16 bis 18 sind zur Illustration. | | | | | |

Es wurden des Weiteren Vergleichsversuche mit Polymerlösungen gemäß Vergleichsbeispielen 1 bis 14 durchgeführt, die entweder nur das Phenoxyethanol a), Ethanol e) oder Isopropanol e) allein oder die Konservierungsmittelmischungen b) und c) als Stabilisator(en) enthalten. Im Vergleich zu den entsprechenden erfindungsgemäßen Beispielen wurde bis zu mehr als die 10-fache Menge an Konservierungsmittel eingesetzt. Hierzu wird auf die folgende Tabelle 2 verwiesen.

**Tabelle 2**

| Vergleichsbeispiel | Polymer in wässriger Polymerlösung | Konservierungsmittel oder Konservierungsmittelmischung | Konzentration der einzelnen Komponenten in der Polymerlsg. (Gew.-%) | Keimbelastungstest | Aussehen der stabilisierten Lösung |
|---|---|---|---|---|---|
| 1 | Luviset Clear * | c) 2-Phenoxyethanol | c) 1,15 | nicht bestanden | sehr leicht trüb |
| 2 | Luviset Clear * | b) Phenylpropanol | b) 0,65 | nicht bestanden | leicht trüb |
| | | 1,2-Octandiol | | | |
| | | 1,3-Propandiol | | | |
| 3 | Luviset Clear * | c) 1,2-Pentandiol | c) 0,75 | nicht bestanden | trüb/Phasentrennung |
| | | 1,2-Octandiol | | | |
| | | 1,2-Decandiol | | | |
| 4 | Luvigel Star ** | b) 2-Phenoyxethanol | c) 1,50 | nicht bestanden | fast klar |
| 5 | Luvigel Star ** | d) Phenylpropanol | c) 0,95 | nicht bestanden | fast klar |
| | | 1,2-Octandiol | | | |
| | | 1,3-Propandiol | | | |
| 6 | Luvigel Star ** | c) 1,2-Pentandiol | d) 1,25 | nicht bestanden | minimal trüb |
| | | 1,2-Octandiol | | | |
| | | 1,2-Decandiol | | | |
| 7 | Luviquat Hold*** | c) Phenoxyethanol | c) 1,15 | nicht bestanden | fast klar/ sehr leicht trüb |
| 8 | Luviquat Hold*** | d) Phenylpropanol | b) 0,6 | nicht bestanden | fast klar/sehr |
| | | 1,2-Octandiol | | | |
| | | 1,3-Propandiol | | | leicht trüb |
| 9 | Luviquat Hold*** | c) 1,2-Pentandiol | c) 0,75 | nicht bestanden | trüb/Phasentrennung |
| | | 1,2-Octandiol | | | |
| | | 1,2-Decandiol | | | |
| 10 | Luviskol K85**** | d) Phenylpropanol | b) 0,65 | nicht bestanden | leicht trüb |
| | | 1,2-Octandiol | | | |
| | | 1,3-Propandiol | | | |
| 11 | Luviskol K90***** | c) Phenoxyethanol | d) 1,25 | nicht bestanden | fast klar |
| 12 | Luviskol K90***** | c) 1,2-Pentandiol | c) 0,65 | nicht bestanden | trüb |
| | | 1,2-Octandiol | | | |
| | | 1,2-Decandiol | | | |
| 13 | Luviskol K90***** | e) Ethanol | e) 3,00 | nicht bestanden | fast klar |
| 14 | Luviskol K90***** | d)Phenoxyethanol Ethylhexylglycerin | d) 1,25 | nicht bestanden | fast klar |

| | | | | | |
|---|---|---|---|---|---|
| * = Copolymer aus Vinylpyrrolidon, Methacrylamid und Vinylimidazol, 20 %ige wässrige Lösung ** = Polyurethan -39, 20 %ige wässrige Lösung *** = Polyquaternium -46, 20 %ige wässrige Lösung **** = Polyvinylpyrrolidon, 20 %ige wässrige Lösung ***** = Polyvinylpyrrolidon, 20 %ige wässrige Lösung | | | | | |

Wie anhand der Vergleichsbeispiele 1 bis 14 zu erkennen ist, haben die mit Phenoxyethanol, Ethanol oder Isopropanol allein oder den Konservierungsmittelmischungen b) und c) allein stabilisierten Polymerlösungen insgesamt den Keimbelastungstest nicht bestanden. Er zeigte nach 7, 14 bzw. 28 Tagen Keimfreiheit im Hinblick auf die getesteten Stämme Pseudomonas aeruginosa (ATCC 9027), Staphylococcus aureus (ATCC 6638) und Candida albicans (ATCC 10231). Allerdings wurde eine Besiedlung mit Aspergillus brasiliensis (ATCC 16404) festgestellt. Außerdem zeigten die wässrigen Vergleichspolymerlösungen eine trübe, gelbliche Färbung, und sie sind inakzeptabel geruchsintensiv. Zudem sind die Konservierungsmittelmischungen b) und c) extrem teuer und damit wenig wirtschaftlich im Vergleich zu den erfindungsgemäßen boosternden Konservierungsmittelmisch ungen.

## Patentansprüche

1. Wässrige 2-Methyl-isothiazolin-3-on-freie Polymerlösung, die mit einer Konservierungsmittelmischung aus Phenoxyethanol und mindestens einem weiteren Alkohol stabilisiert ist, wobei das Polymer ausgewählt ist aus Vinyllactam-Polymeren und Polyurethanen, und wobei entweder die Konservierungsmittelmischung als biozide Wirkstoffe folgende Bestandteile aufweist:
- 25 bis 50 Gew.-% einer Mischung aus 50% 3- Phenylpropanol, 25% 1,3-Propandiol und 25% 1,2-Octandiol und
- 50 bis 75 Gew.-% Phenoxyethanol,
und dann
in einer Menge von 0,10 bis 1,40 Gew.-% in der wässrigen Polymerlösung eingesetzt wird,
oder die
Konservierungsmittelmischung als biozide Wirkstoffe folgende Bestandteile aufweist:
- 20 bis 57 Gew.-% einer Mischung aus 50% 1,2-Pentandiol, 30% 1,2-Octandiol und 20% 1,2-Decandiol und
- 43 bis 80 Gew.-% Phenoxyethanol,
und dann
in einer Menge von 0,20 bis 1,40 Gew.-% in der wässrigen Polymerlösung eingesetzt wird.

2. Verwendung der wässrigen Polymerlösung nach Anspruch 1 in kosmetischen Zubereitungen und Produkten für den Home Care Bereich.

3. Verwendung nach Anspruch 2 in Haut- und Haarpflegemitteln.

4. Verwendung der wässrigen Polymerlösung nach Anspruch 1 in Haarstylingprodukten, in Produkten der dekorativen Kosmetik und Haar- und Körperreinigung.

## Claims

1. An aqueous 2-methylisothiazolin-3-one-free polymer solution which has been stabilized with a preservative mixture composed of phenoxyethanol and at least one further alcohol, wherein the polymer is selected from vinyl lactam polymers and polyurethanes, and wherein either the preservative mixture comprises the following constituents as active biocidal ingredients:
- from 25 to 50% by weight of a mixture composed of 50% 3-phenylpropanol, 25% 1,3-propanediol and 25% 1,2-octanediol and
- from 50 to 75% by weight of phenoxyethanol,
and is then
used in an amount of from 0.10 to 1.40% by weight in the aqueous polymer solution,
or the
preservative mixture comprises the following constituents as active biocidal ingredients:
- from 20 to 57% by weight of a mixture composed of 50% 1,2-pentanediol, 30% 1,2-octanediol and 20% 1,2-decanediol and
- from 43 to 80% by weight of phenoxyethanol,
and is then
used in an amount of from 0.20 to 1.40% by weight in the aqueous polymer solution.

2. The use of the aqueous polymer solution according to claim 1 in cosmetic preparations and products for the home care sector.

3. The use according to claim 2 in skincare and haircare products.

4. The use of the aqueous polymer solution according to claim 1 in hairstyling products, in products for decorative cosmetics and hair and body cleansing.

## Revendications

1. Solution aqueuse de polymère exempte de 2-méthyl-isothiazolin-3-one, qui est stabilisée par un mélange de conservateurs composé de phénoxyéthanol et d'au moins un autre alcool, le polymère étant choisi parmi des polymères de vinyllactame et des polyuréthanes, et soit le mélange de conservateurs présente les ingrédients suivants en tant que principes actifs biocides :
- 25 à 50 % en poids d'un mélange composé de 50 % de 3-phénylpropanol, 25 % de 1,3-propanediol et 25 % de 1,2-octanediol et
- 50 à 75 % en poids de phénoxyéthanol,
et ensuite
est utilisé en une quantité de 0,10 à 1,40 % en poids dans la solution aqueuse de polymère,
soit le mélange de conservateurs présente les ingrédients suivants en tant que principes actifs biocides :
- 20 à 57 % en poids d'un mélange de 50 % de 1,2-pentanediol, 30 % de 1,2-octanediol et 20 % de 1,2-décanediol et
- 43 à 80 % en poids de phénoxyéthanol,
et ensuite
est utilisé en une quantité de 0,20 à 1,40 % en poids dans la solution aqueuse de polymère.

2. Utilisation de la solution aqueuse de polymère selon la revendication 1 dans des préparations cosmétiques et des produits pour le domaine d'entretien domestique.

3. Utilisation selon la revendication 2 dans des produits de soins de la peau et capillaires.

4. Utilisation de la solution aqueuse de polymère selon la revendication 1 dans des produits de coiffure, dans des produits cosmétiques décoratifs et de nettoyage capillaire et corporel.
